# EUROPEAN PATENT SPECIFICATION

(11) **EP 2 686 445 B1**
(45) Date of publication and mention of the grant of the patent: **17.05.2017**
(21) Application number: 12757678.3
(22) Date of filing: 13.03.2012
(51) Int. Cl.: C12Q 1/68, C12N 15/11, G01N 33/52

(54) **METHOD OF IDENTIFYING NUCLEIC ACID-CONTAINING OBJECT**
VERFAHREN ZUR IDENTIFIKATION NUKLEINSÄUREHALTIGER OBJEKTE
PROCÉDÉ D'IDENTIFICATION D'UN OBJET CONTENANT DES ACIDES NUCLÉIQUES

(30) Priority: 14.03.2011 KR 20110022378
(43) Date of publication of application: 22.01.2014
(73) Proprietor: Bioneer Corporation, Daejeon 306-220 (KR)
(72) Inventor: PARK, Han Oh, Daejeon 305-761 (KR); CHOI, Jung Young, Daejeon 305-506 (KR); HAN, Eun Su, Daejeon 305-791 (KR); SONG, Gu Young, Daejeon 305-762 (KR); JANG, Won Seok, Daejeon 305-751 (KR)
(74) Representative: Beck Greener
(86) International application number: PCT/KR2012/001815
(87) International publication number: WO 2012/124968

(56) References cited:
- WO-A2-00/61799
- WO-A2-2007/103558
- JP-A- 2006 191 856
- RIZZO J ET AL: "Chimeric RNA-DNA molecular beacon assay for ribonuclease H activity", MOLECULAR AND CELLULAR PROBES, ACADEMIC PRESS, LONDON, GB, vol. 16, no. 4, 1 August 2002 (2002-08-01) , pages 277-283, XP002436558, ISSN: 0890-8508, DOI: 10.1006/MCPR.2002.0423
- GERASIMOVA, Y. V. ET AL.: 'A single molecular beacon probe is sufficient for the analysis of multiple nucleic acid sequences' CHEMBIOCHEM. vol. 11, no. 12, 16 August 2010, pages 1762 - 1768, XP055123924
- HOSODA, K. ET AL.: 'A novel sequence-specific RNA quantification method using nicking endonuclease, dual-labeled fluorescent DNA probe, and conformation-interchangeable oligo-DNA' RNA vol. 14, no. 3, March 2008, pages 584 - 592, XP055098081
- YANG, C. J. ET AL.: 'Monitoring nucleic acids using molecular beacons' CURR. PHARM. BIOTECHNOL. vol. 6, no. 6, December 2005, pages 445 - 452, XP008170706
- JOHANSSON, M. K. ET AL.: 'Choosing reporter-quencher pairs for efficient quenching through formation of intramolecular dimers' METHODS MOL. BIOL. vol. 335, 2006, pages 17 - 29, XP008170702

<110> BIONEER CORPORATION
<120> METHOD OF IDENTIFYING NUCLEIC ACID-CONTAINING OBJECT
<130> 2012-opa-5249
<150> KR 10-2011-0022378
   <151> 2011-03-14
<160> 2
<170> KopatentIn 2.0
<210> 1
   <211> 10
   <212> DNA
   <213> Artificial Sequence
<220>
   <223> oligonucleotide sequence
<400> 1
   accgtgacgt 10
<210> 2
   <211> 10
   <212> RNA
   <213> Artificial Sequence
<220>
   <223> RNA dual-probe sequence complementary to the oligonucleotide of SEQ. ID. NO: 1
<400> 2
   acgucacggu 10

## Description

### Technical Field

The present invention relates to a method of identifying nucleic acid-containing object, more precisely a method of identifying nucleic acid-containing object having the nucleotide sequence recognized by ribonuclease (RNase) easily using fluorescence.

### Background Art

Nucleic acid such as oligonucleotide can be amplified at a large scale via PCR (polymerase chain reaction) even though it is included in a sample at an extremely low concentration. And such nucleic acid included with a very small amount can be identified by analyzing the nucleotide sequence. Thus, the source and the rout of transportation of an object or a product can be accurately tracked down and the appraisal of genuine product can also be accurately performed by adding a minimum amount of the nucleic acid to a target object or product including oil products such as oil or paint, gunpowder, and valuable works of arts, etc. The previous articles reporting the use of such oligonucleotide for investigating transportation route of a product are as follows:

US Patent No. 5,665,538 describes a method for monitoring the movement of petroleum in aqueous solution. Precisely in this description, it is explained that DNA is added to petroleum as a microtrace additive and then sampling of the petroleum containing the DNA microtrace additive is performed after the movement of petroleum, followed by PCR to detect the DNA microtrace additive. US Patent No. 5,451,505 describes a method for monitoring an object exposed on natural UV. Precisely, nucleic acid is collected, followed by PCR to amplify the nucleic acid in order to confirm the target object. In the meantime, EP 1171633 describes a method for quantitative detection of nucleic acid by real-time PCR using a primer and a fluorescence-labeled probe as a nucleic acid tag. However, such conventional methods have disadvantages, for example they do not facilitate quantitative analysis of a sample at a very low concentration; it is hard to label the product differently; there is a risk of contamination or manipulation such as elimination, etc by a handler; and it is very difficult to commercialize because it takes a very long time to detect such nucleic acid.

To overcome the limitations of the prior arts, the oligonucleotide with improved solubility to lipophilic solvent and a detection method of a target object using the oligonucleotide has been developed (Korean Patent No. 0851764) along with the oligonucleotide marker appropriate for being used as a recognition label or marker for a vehicle when it is added to automobile paint film and a detection method of a vehicle using the same (Korean Patent No. 0851765). According to the method, the oligonucleotide dissolved in paint, at a small concentration, is extracted and recovered, followed by PCR to amplify the nucleotide sequence. By sequencing the nucleotide sequence, a target subject can be traced and confirmed. However, in this method, sequence information is coded, so that the process of decoding the coded sequence is required. In addition, for the sequencing, costs, accuracy, processing time, and complication of the process are also problems which make commercialization difficult. That is, the methods have problems in fast and simple judgment of authentic object and in confirmation of coded information (lot no., unique recognition number of the manufacturer, etc). According to the recent trend providing a variety oil products, manipulation of oil grade and distribution of illegal gasoline have been social issues. Therefore, it is required to develop a faster and simpler efficient method to identify and recognize the kind and quality of oil products on the market for the brand-image management of a manufacturer and for the establishment of distribution order.

### Disclosure of Invention

### Technical Problem

The present invention has been designed to improve the problems of the conventional method for recognition of an object using nucleic acid. Therefore, it is an object of the present invention to provide a method of identifying a nucleic acid marker labeled instantly by using nucleic acid recovered from the nucleic acid labeled object.

### Solution to Problem

The method of identifying nucleic acid-containing object comprises the following steps:
(1) preparing nucleic acid-containing object having the nucleotide sequence complementary to the nucleotide sequence of RNA-dual probe;
(2) reacting the nucleic acid included in the object with the buffer containing the RNA-dual probe conjugated with a reporter and a quencher respectively and RNase; and
(3) detecting fluorescence generated from the reporter.

In this description, the term "RNA-dual probe" indicates the dual-labeled RNA probe having the structure in which a reporter and a quencher are bound to RNA probe respectively. The reporter and quencher can be conjugated with any site of the RNA probe, but more preferably can be conjugated with both ends of the RNA.

In step (1), the nucleic acid can be any single-stranded or double-stranded DNA, RNA, or DNA/RNA hybrid, and more preferably can be single-stranded DNA, but not always limited thereto. The nucleic acid-containing object herein is preferably liquid, which is exemplified by petroleum oil such as gasoline, kerosene, and diesel, paint for automobile coating, lacquer, paint for traffic lane indication, paint diluent, thinner, gunpowder, natural oil, paint for construction, organic solvent, glue, dye, meat, and sea food, but not always limited thereto. The nucleic acid is preferably oligonucleotide having 5 - 1,000 nucleotides, and more preferably having 10 - 100 nucleotides, but not always limited thereto. RNA is comparatively easily degraded. So, when DNA is included in the object, stability increases, which is more preferred.

In a preferred embodiment of the present invention, the nucleic acid can be conjugated with a quaternary ammonium salt compound or a cationic surfactant, which is a cationic phase transfer agent. In this invention, the cationic phase transfer agent is quaternary alkyl ammonium ion, which can be exemplified by tetrabutyl ammonium hydroxide or hexadecyl trimethyl ammonium bromide. The nucleic acid is preferably blocked by a blocking agent, and the blocking agent is characteristically lipid or phosphate or the chemical not having terminal group.

In this disclosure nitrogen and oxygen sites (reactive region) of the nucleic acid are characteristically conjugated with the C₁-C₅₀ organic compound. The C₁-C₅₀ organic compound can be exemplified by carbonyl compounds forming amide bond with nitrogen part and forming ester bond with oxygen part, silanyl compounds forming O-Si bond with N-Si, sulfonyl compounds forming O-S bond with NS, saturated hydrocarbons forming O-C bond with N-C which is breakable when ammonia is treated, aromatic hydrocarbons, unsaturated hydrocarbons, saturated or unsaturated hydrocarbons containing heteroatom, etc.

In the meantime, there is no limitation in the method for marking or recovering the nucleic acid to or from the object, and thus any conventional method can be used. In a preferred embodiment of the present disclosure the nucleic acid can be prepared by adding lipid to 3'-end and/or 5'-end of the nucleic acid. The lipid can be C₃-C₁₀₀ lipids, preferably C₇-C₂₄ lipids,withoutlimitation. In a preferred embodiment of the present invention, the nucleic acid can be prepared by adding C₁₂ lipid to 3'-end and C₁₈ lipid to 5'-end. The nucleic acid can be dissolved in petroleum oil, preferably in gasoline, but not always limited thereto. At this time, a cationic phase transfer agent that can be bound with anionic site of the nucleic acid by electrostatic attraction can be added to the nucleic acid before experiment. In another preferred embodiment of the present invention, the nucleic acid can be recovered from the object by making it be conjugated with the cationic phase transfer agent by adding an anionic phase transfer agent (-PTA) that can be a count-ion of the cationic phase transfer agent (+PTA). At this time, the anionic phase transfer agent (-PTA) can be the count-ion of the cationic phase transfer agent (+PTA) which is bound to the nucleic acid in the organic solvent by electrostatic attraction, or any reagent that can be dissolved in the organic solvent can be used without limitation.

In step (2), the reporter and the quencher can be conjugated with either 5'-end or 3'-end of the RNA-dual probe respectively. The reporter is preferably TAMRA (Carboxy-tetramethyl-hod-amine), FAM (6-carboxyfluorescein), Cy3, Cy5, or Cy5, while the quencher is preferably BHQ1 (2,5-di-tert- butylhydroquinone-1), BHQ2, TAMRA, or DABCYL, but not always limited thereto and in fact a variety of fluorescent materials can be used or any combination of them can be used. The buffer herein is not limited, and any buffer having the conventional composition that is appropriate for the amplification of nucleic acid or for the biochemical reaction of nucleic acid can be used. However, it is more preferred to use the buffer containing one or more ingredients selected from the group consisting of Tris, KCl, MgCl₂, MnCl₂ , and Dithiothreitol. The buffer herein can selectively include RNase inhibitor.

RNase is the enzyme that breaks down RNA, which is classified into two types such as endo-type that digests the middle part of the nucleotide sequence and exo-type that cuts the end of the nucleotide sequence. RNase has a wide range of substrate specificity and is involved in a very complicated physiological activity. In this invention, RNase can be any random RNase that can digest RNA part of the conjugate in which nucleic acid of the object is conjugated with RNA-dual probe, and is more preferably RNase H. RNase H is the enzyme that specifically digests RNA alone from single-stranded DNA/single-stranded RNA hybrid, which was first separated by W. H. Stein and P. Hausen in 1969 from calf thymus (Stein H, Hausen P., Science, 1969, 166(903), 393-395). This enzyme is found in various eukaryotic cells such as animal cells and yeast and prokaryotic cells such as *E.coli.* RNase H is the endo-type enzyme showing substrate specificity and particularly digesting RNA strand only from DNA/ RNA hybrid. This enzyme needs divalent metal ions such as Mg²⁺ and Mn²⁺ for its activation. In a preferred embodiment of the present invention, the RNase H has its activity at the temperature range of 20°C - 90°C.

In step (3), the detection of fluorescence is performed by using a proper detection device to detect fluorescence of a specific wavelength band generated from the reporter. The wavelength herein is varied with the kind of the reporter and is not limited to a specific wavelength band.

In a preferred embodiment of the present invention, nucleic acid was recovered from the nucleic acid-containing object and then reacted with the buffer containing RNA-dual probe and RNase. Precisely, the method of identifying nucleic acid-containing object of the present disclosure comprises the following steps:
(1) preparing nucleic acid-containing object having the nucleotide sequence complementary to the nucleotide sequence of RNA-dual probe;
(2) recovering the nucleic acid from the object;
(3) reacting the nucleic acid included in the object with the buffer containing the RNA-dual probe conjugated with a reporter and a quencher respectively and RNase; and
(4) detecting fluorescence generated from the reporter.

In a preferred embodiment of the present invention, it was easily confirmed whether or not the target object contained nucleic acid by measuring fluorescence after reacting the oligonucleotide recovered from the solution containing the oligonucleotide in the reaction buffer comprising RNA-dual probe and RNase H for a required time, preferably for 1 minute - 1 hour, more preferably for 5 minutes - 10 minutes. According to this method, there was no step of purifying DNA. So, the possibility of incorrect fluorescence signal detection caused by cross-contamination by cells or other genomic DNA could be actually eliminated.

In another preferred embodiment of the present invention, the method of the invention was confirmed to facilitate the verification of distribution channel of a product, place of production, and discrimination of a genuine article by measuring fluorescence generated from the reporter. Precisely, to identify products each containing different nucleic acid, the nucleic acid included in each product was reacted with RNA-dual probe which was bound complementarily to the nucleic acid of the product, followed by detection of fluorescence. As a result, it could be easily determined which product contained which nucleic acid.

In another preferred embodiment of the present invention, it was easily confirmed whether the specific product, for example oil products, was genuine or not by the method of the invention. For example, there was a similar product in which a genuine oil product was mixed with an illegal oil product at the ratio of 1:1. At this time, if considered fluorescence measured from the genuine oil product as 100, the fluorescence level of the similar product would be about 50. Based on the principal, distribution channel of oil products, discrimination of genuineness, and inclusion ratio of the genuine product could be easily confirmed.

### Advantageous Effects of Invention

According to the method of the present invention, even if the marker oligonucleotide is included in the target object at an extremely small amount, it can be identified with high accuracy within a short period of time, for example within 10 minutes. In this invention, the size or the nucleotide sequence of oligonucleotide can be varied, suggesting that a variety of labeling can be made and labeling sensitivity can also be increased by the numbers of different combinations of various fluorescent dyes. In addition, the method of identifying a nucleic acid-containing object of the present invention provides labeling sensitivity 100 times as high as the conventional method using sequencing or labeling with fluorescent materials. Besides, the method of the present invention is characterized by shorter analyzing time, facilitates different labeling on a variety of products according to fluorescent materials, and makes possible unlimited product administration by product group and batch in real production process by differentiating the nucleotide sequence of each oligonucleotide.

### Brief Description of Drawings

The application of the preferred embodiments of the present invention is best understood with reference to the accompanying drawings, wherein:
Figure 1 is a graph illustrating the measurement of emission wavelength in the experimental group added with RNase H.
Figure 2 is a graph illustrating the increase of emission value dose-dependently as the content of RNA-dual probe increases.
Figure 3 is a graph illustrating the increase of emission value as the content of RNase H increases.
Figure 4 is a graph illustrating the measurement of fluorescence as RNase reaction proceeds at a fixed temperature.
Figure 5 is a set of graphs illustrating the real-time measurement of fluorescence generated by RNase reaction regardless of types of fluorescent materials. In Figure 5A, TAMRA and BHQ2 were used respectively as a reporter and a quencher. In Figure 5B, FAM and BHQ1 were used respectively as a reporter and a quencher. In Figure 5C, Cy3 and BHQ2 were used respectively as a reporter and a quencher. In Figure 5D, Cy5 and BHQ2 were used respectively as a reporter and a quencher. Figure 5E is a graph exhibiting the graphs of Figure 5A - Figure 5D all at once.
Figure 6 is a graph illustrating the measurement of fluorescence generated by RNase activity even with less than 10 minute measurement time.

### Best Mode for Carrying out the Invention

Practical and presently preferred embodiments of the present invention are illustrative as shown in the following Examples.

However, it will be appreciated that those skilled in the art, on consideration of this disclosure, may make modifications and improvements within the scope of the present invention.

### Example 1: Preparation of the nucleic acid-containing object having the nucleotide sequenceu binding complementarily to RNA-dual probe

### <1-1> Preparation of oligonucleotide

Oligonucleotide having the nucleotide sequence represented by SEQ. ID. NO: 1 corresponding to the 10 mer template DNA was synthesized on the surface of an immobilized carrier (CPG) for oligonucleotide synthesis by using an automatic sequencer.

5'-ACCGTGACGT-3', MW=3027.96 (SEQ. ID. NO: 1)

The oligonucleotide having the nucleotide sequence represented by SEQ. ID. NO: 1 used as a template can be synthesized with the addition of lipid on both ends. In this example, C₁₂ lipid was added to 3'-end and C₁₈ lipid was added to 5'-end to prepare the oligonucleotide of the invention.

5'-C18-ACCGTGACGT-C12-3'

The synthesized oligonucleotide was recovered from the immobilized carrier (CPG) by using ammonia water (concentration of ammonia: 28%). Particularly, 1 mℓ of 28 weight% ammonia water was added to the immobilized carrier (CPG 10 mg), which was air-tight sealed, followed by reaction at 65°C for 3 hours. Then, the oligonucleotide in solution was obtained by recovering the ammonia water. The oligonucleotide was purified by BioRP (Bioneer), the oligonucleotide purification method. The oligonucleotide in solution was quantified by measuring UV absorption (260 nm).

### <1-2> Preparation of gasoline containing oligonucleotide

The oligonucleotide prepared by adding C₁₂ lipid to its 3'-end and C₁₈ lipid to its 5'-end in Example 1 was dissolved in distilled water. The concentration was adjusted to 50 OD/mℓ. The oligonucleotide dissolved in distilled water was loaded in 15 mℓ conical tube (Corning) at the concentration of 100 OD. As a phase transfer agent (PTA), hex-adecyltrimethylammonuim bromide (MW=364.5), the cationic phase transfer agent (+PTA) which is possibly linked to anionic site of the oligonucleotide by electrostatic attraction was diluted in distilled water at the concentration of 1.3 mM. It was added to the oligonucleotide to make the total volume 3 mℓ. Same volume of gasoline (SK Oil Refinery Co.) was added thereto. The mixture was well mixed in vortex at least 1 minutes, during which polar site of the oligonucleotide in solution is bound to the phase transfer agent by electrostatic attraction. As a result, the oligonucleotide was neutralized and dissolved in gasoline.

### <1-3> Recovery of nucleic acid from the object

Since the oligonucleotide was stably dissolved in gasoline, it was hardly dissolved in the water layer simply by mixing water or boiling after adding ammonia water. Thus, an anionic phase transfer agent (-PTA) acting as the count-ion to the cationic phase transfer agent (+PTA) was added thereto to make the count-ion be conjugated with the cationic phase transfer agent instead of being conjugated with the oligonucleotide. As a result, the oligonucleotide could be extracted by using water. Particularly, HDEHP (Bis(2-ethylhexyl) phosphate, M.W:322.42) dissolved in sterilized water at the concentration of 0.5 M was added to the oligonucleotide/gasoline mixture prepared in Example <1-2>. The mixture was centrifuged at 3,000 rpm for 10 minutes to separate water layer and organic solvent layer. The aqueous solution stayed at the lower part was taken and UV absorption was measured. Then, the oligonucleotide remaining in water layer was recovered.

### Example 2: Light emission by RNase H

It was investigated whether or not light emission by RNase H was observed from the oligonucleotide recovered in Example <1-3> and RNA-dual probe. The oligonucleotide having the nucleotide sequence represented by SEQ. ID. NO: 1 added with C₁₂ lipid at 3'-end and C₁₈ lipid at 5'-end, recovered in Example <1-3>, and the RNA-dual probe having the nucleotide sequence represented by SEQ. ID. NO: 2 complementary to the oligonucleotide were synthesized (see Table 1). The RNA-dual probe used in this invention was in the length of 10 mer, whose 5'-end was labeled with the fluorescent material reporter TAMRA and 3'-end was labeled with the quencher BHQ2.

Fluorescence emission by RNase H was confirmed by the following steps. RNase H reaction to the nucleotide/RNA-dual probe conjugate was confirmed at a certain temperature by using a gene amplification apparatus (MyGenie96; Bioneer). And fluorescence emission by RNase H was also confirmed by using a spectrofluorophotometer (SHIMADZU). At this time, the sample not containing RNase H was used as the control.
(1) Total 20 µℓ of sample was prepared as shown in Table 2.
(2) Reaction was induced in a gene amplification apparatus (MyGenie 96) by raising the temperature from 20°C to 90°C by 0.5°C/sec.
(3) Upon completion of the reaction, the reaction mixture was mixed with 1,980 mℓ of 1 M Tris-HCl, followed by measuring fluorescence wavelength with a spectrofluorophotometer (SHIMADZU).
Table 1

**[Table 1]**

| | Nucleotide sequence (5'→3') | SEQ. ID. NO: |
|---|---|---|
| Template DNA sequence | ACCGTGACGT | 1 |
| Oligonucleeotide | C18-ACCGTGACGT-C12 | 1 |
| RNA dual-probe | TAMRA-ACGUCACGGU-BHQ2 | 2 |

Table 2

**[Table 2]**

| Upon 1 tube reaction | |
|---|---|
| Oligonucleotide | 1 pmole/*µ*ℓ |
| RNA-dual probe | 100 pmole/*µ*ℓ |
| RNase H | 5 unit/*µ*ℓ |
| Buffer solution | to make final 1x |
| DEPC-distilled water | to make final volume 20 *µ*ℓ |
| Total volume | 20 *µ*ℓ |

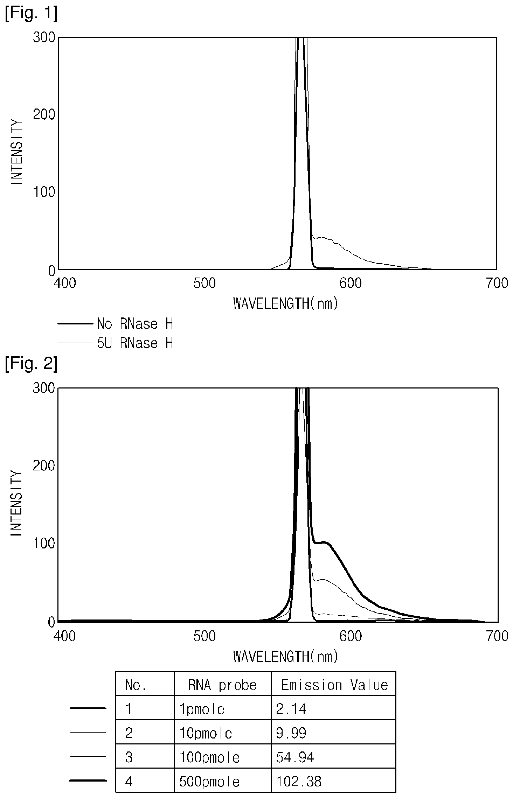

As a result, it was confirmed that emitting wavelength was measured in the experimental group added with 5 unit/µℓ of RNase H (Figure 1).

### Example 3: Cycle reaction and RNA-dual probe dependent emission

Reaction was induced with changing the reaction temperature of the oligonucleotide recovered in Example <1-3> and the RNA-dual probe and the reaction temperature of RNase H by using a gene amplification apparatus (MyGenie96; Bioneer). This reaction was repeated 40 cycles and then the increase of fluorescence level was confirmed. Fluorescence emission by RNase H was also confirmed by using a spectrofluorophotometer. At this time, different concentrations of the RNA-dual probe, 1, 10, 100, and 500 pmole/µℓ, were independently used.
(1) Total 20 µℓ of sample was prepared as shown in Table 3.
(2) Reaction was induced in a gene amplification apparatus (MyGenie 96) at denaturation temperature, reaction temperature, and RNase H reaction temperature as shown in Table 4 in that order.
(3) Upon completion of the reaction, the reaction mixture was mixed with 1,980 mℓ of 1 M Tris-HCl, followed by measuring fluorescence wavelength with a spectrofluorophotometer (SHIMADZU).
Table 3

**[Table 3]**

| Upon 1 tube reaction | |
|---|---|
| Oligonucleotide | 1 pmole/*µ*ℓ |
| RNA-dual probe | 1, 10, 100, 500 pmole/*µ*ℓ |
| RNase H | 5 unit/*µ*ℓ |
| Buffer solution | to make final 1x |
| DEPC-distilled water | to make final volume 20 *µ*ℓ |
| Total volume | 20 *µ*ℓ |

Table 4

**[Table 4]**

| Reaction | Temperature (time) | Cycles |
|---|---|---|
| Denaturation | 40°C (1 minute) | 1 cycle |
| Reaction temperature | 25°C (10 seconds) | 40 cycles |
| RNase H reaction temperature | 37°C (10 seconds) | |

As a result, it was confirmed that the emission level was increased as the content of the RNA-dual probe was increased from 1 pmole to 500 pmole, dose-dependently (Figure 2).

### Example 4: Measurement of RNase H activity by using real-time PCR machine

To confirm the RNase H activity by real-time, reaction was performed by the following procedure using Exicycler (Bioneer), the real-time PCR machine. At this time, RNase H was independently used at different concentrations of 1, 5, and 10 unit/ µℓ and the oligonucleotide recovered in Example <1-3> was used.
(1) Total 20 µℓ of sample was prepared as shown in Table 5.
(2) Reaction was induced in a real-time PCR machine at denaturation temperature, reaction temperature, and RNase H reaction temperature as shown in Table 6 in that order.
(3) Upon completion of the reaction, the reaction mixture was mixed with 1,980 mℓ of 1 M Tris-HCl, followed by measuring fluorescence wavelength with a spectrofluorophotometer (SHIMADZU).
Table 5

**[Table 5]**

| Upon 1 tube reaction | |
|---|---|
| Oligonucleotide | 1 pmole/*µ*ℓ |
| RNA-dual probe | 100 pmole/*µ*ℓ |
| RNase H | 1,5, 10 unit/*µ*ℓ |
| Buffer solution | to make final 1x |
| DEPC-distilled water | to make final volume 20 *µ*ℓ |
| Total volume | 20 *µ*ℓ |

Table 6

**[Table 6]**

| | | |
|---|---|---|
| Reaction | Temperature (time) | Cycles |
| Denaturation | 40°C (1 minute) | 1 cycle |
| Reaction temperature | 25°C (10 seconds) | 40 cycles |
| RNase H reaction temperature | 37°C (10 seconds) | |

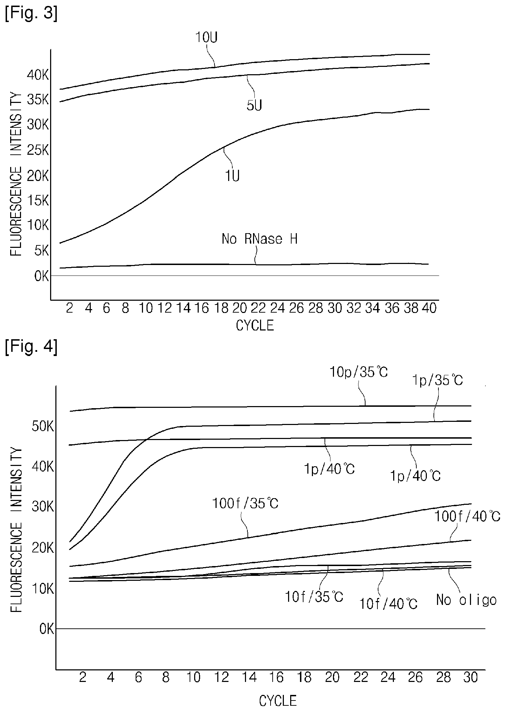

As a result, it was confirmed that the emission level was increased as the unit of RNase H added to the reaction mixture increased (Figure 3).

### Example 5: Real-time activity according to the fixed reaction temperature

As shown in Table 7, total 20 µℓ of sample was prepared. Temperature of reaction between the oligonucleotide and the RNA-dual probe was set as 35°C or 40°C. Fluorescence level was measured by using a real-time PCR machine. Fluorescence emission by RNase H was confirmed every minute by using a spectrofluorophotometer. At this time, the oligonucleotide recovered in Example <1-3> was used at different concentrations of 10 fmole/µℓ, 100 fmole/µℓ, 1 pmole/µℓ, and 10 pmole/ µℓ.
Table 7

**[Table 7]**

| Upon 1 tube reaction | |
|---|---|
| Oligonucleotide | 10 fmole/*µ*ℓ, 100 fmole/*µ*ℓ, 1 pmole/*µ*ℓ and 10 pmole/*µ*ℓ |
| RNA-dual probe | 100 pmole/*µ*ℓ |
| RNase H | 2 unit/*µ*ℓ |
| Buffer solution | to make final 1x |
| DEPC-distilled water | to make final volume 20 *µ*ℓ |
| Total volume | 20 *µ*ℓ |

As a result, fluorescence was detected, suggesting that RNase reaction was successfully progressed even at the fixed temperature (Figure 4).

### Example 6: Real-time activity according to the fluorescent material

The nucleotide sequence shown in Table 1 was conjugated with different RNA-dual probes of TAMRA-BHQ2, FAM-BHQ1, Cy3-BHQ2, or Cy5-BHQ2. As shown in Table 8, total 20 µℓ of sample was prepared. Increase of fluorescence level was confirmed by using a real-time PCR machine under the reaction conditions as shown in Table 9. Fluorescence emission by RNase H was confirmed by using a spectrofluorophotometer. At this time, the oligonucleotide recovered in Example <1-3> was used at different concentrations of 100 fmole/µℓ, and 1 pmole/µℓ, independently. Reaction was induced according to the conditions shown in Table 9 and fluorescence level was measured by real-time even at different wavelengths.
Table 8

**[Table 8]**

| Upon 1 tube reaction | |
|---|---|
| Oligonucleotide | 100 fmole/*µ*ℓ, 1 pmole/*µ*ℓ |
| RNA-dual probe | 100 pmole/*µ*ℓ |
| RNase H | 2 unit/*µ*ℓ |
| Buffer solution | to make final 1x |
| DEPC-distilled water | to make final volume 20 *µ*ℓ |
| Total volume | 20 *µ*ℓ |

Table 9

**[Table 9]**

| Reaction | Temperature (time) | Cycles |
|---|---|---|
| Reaction temperature & RNase H reaction temperature | 35°C (1 minute) | 12 cycles |

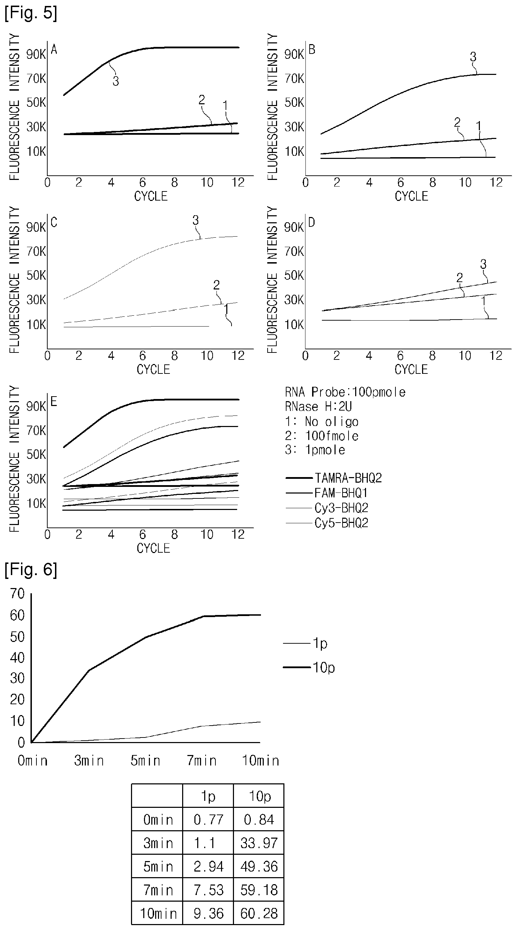

As a result, it was confirmed that even though fluorescence level was varied with each fluorescent material, fluorescence generated by RNase reaction could be detected by real-time regardless of the kind of the fluorescent material (Figure 5).

### Example 7: Measurement of real-time activity using portable fluorometer

As shown in Table 10, total 20 µℓ of sample was prepared. Reaction was performed by using a gene amplification apparatus (MyGenie 96) at 35°C for 0 - 10 minutes, as shown in Table 11. Fluorescence level was measured by using a portable fluorometer (BioQ™-*mini*Fluorometer,Bioneer). At this time, the oligonucleotide recovered in Example <1-3> was used at different concentrations of 1 fmole/µℓ, and 10 pmole/µℓ, independently.
Table 10

**[Table 10]**

| Upon 1 tube reaction | |
|---|---|
| Oligonucleotide | 1 pmole/µℓ, 10 pmole/µℓ |
| RNA-dual probe | 100 pmole/µℓ |
| RNase H | 2 unit/µℓ |
| Buffer solution | to make final 1x |
| DEPC-distilled water | to make final volume 20 µℓ |
| Total volume | 20 µℓ |

Table 11

**[Table 11]**

| Reaction | Temperature (time) |
|---|---|
| Reaction Temperature & RNase H reaction temperature | 35°C (0 - 10 minutes) |
| RNase H inactivation | 90°C (5 minutes) |

As a result, it was confirmed that fluorescence generated by RNase H activity could be measured by even with short period of time of less than 10 minutes (Figure 6).

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the scope of the invention as set forth in the appended claims.
## Claims

1. A method of identifying nucleic acid-containing object, comprising the following steps:
(1) preparing nucleic acid-containing object having the nucleotide sequence complementarily to the nucleotide sequence of RNA-dual probe;
(2) reacting the nucleic acid included in the object with the buffer containing the RNA-dual probe conjugated with a reporter and a quencher respectively and RNase; and
(3) detecting fluorescence generated from the reporter,
wherein the nucleic acid of step (1) is conjugated with a cationic phase transfer agent, and
wherein the nucleic acid has the addition of lipid at 3'-end and/or at 5'-end.

2. The method according to claim 1, wherein the nucleic acid is selected from the group consisting of DNA, RNA, and DNA-RNA hybrid.

3. The method according to claim 1 or claim 2, wherein the nucleic acid is single-stranded DNA.

4. The method according to claim 1, wherein the lipid is C₇ - C₂₄ lipid.

5. The method according to claim 1, wherein the nucleic acid-containing object is selected from the group consisting of gasoline, kerosene, diesel, paint for automobile coating, lacquer, paint for traffic lane indication, paint diluent, thinner, gunpowder, natural oil, paint for construction, organic solvent, glue, dye, meat, and sea food.

6. The method according to claim 1, wherein the nucleic acid has the nucleotide sequence comprising 5 - 1,000 nucleotides.

7. The method according to claim 1, wherein the RNase is RNase H.

8. The method according to claim 1, wherein the reporter and the quencher are conjugated with either 5'-end or 3'-end of the RNA-dual probe.

9. The method according to claim 8, wherein the reporter is selected from the group consisting of TAMRA (Carboxy-tetramethyl-hod-amine), FAM (6-carboxyfluorescein), Cy3, Cy5, and Cy5.5.

10. The method according to claim 8, wherein the quencher is selected form the group consisting of BHQ1 (2,5-di-tert-butylhydroquinone-1), BHQ2, TAMRA, and DABCYL.

11. A method of identifying nucleic-acid containing object, comprising the following steps:
(1) preparing nucleic-acid containing object having the nucleotide sequence complementarily to the nucleotide sequence of RNA-dual probe;
(2) recovering the nucleic acid from the object;
(3) reacting the nucleic acid recovered in step (2) with the buffer containing the RNA-dual probe conjugated with a reporter and a quencher respectively and RNase; and
(4) detecting fluorescence generated from the reporter,
wherein the nucleic acid of step (1) is conjugated with a cationic phase transfer agent, and
wherein the nucleic acid has the addition of lipid at 3'-end and/or at 5'-end.

12. The method according to claim 1, wherein the buffer additionally contains RNase inhibitor.

13. A method of confirming whether a nucleic acid-containing object is genuine, comprising the following steps:
(1) preparing nucleic acid-containing object having the nucleotide sequence complementarily to the nucleotide sequence of RNA-dual probe;
(2) reacting the nucleic acid included in the object with the buffer containing the RNA-dual probe conjugated with a reporter and a quencher respectively and RNase; and
(3) detecting fluorescence generated from the reporter,
wherein the nucleic acid of step (1) is conjugated with a cationic phase transfer agent, and
wherein the nucleic acid has the addition of lipid at 3'-end and/or at 5'-end.

14. The method according to claim 13, wherein the nucleic acid-containing object is selected from the group consisting of gasoline, kerosene, diesel, paint for automobile coating, lacquer, paint for traffic lane indication, paint diluent, thinner, gunpowder, natural oil, paint for construction, organic solvent, glue, dye, meat, and sea food.

## Patentansprüche

1. Verfahren zum Identifizieren eines Nukleinsäure enthaltenden Objekts, das die folgenden Schritte beinhaltet:
(1) Vorbereiten des Nukleinsäure enthaltenden Objekts, mit der Nukleotidsequenz komplementär zur Nukleotidsequenz für RNA-Dual-Sonde;
(2) Reagieren der im Objekt eingeschlossenen Nukleinsäure mit dem Puffer, die RNA-Dual-Sonde enthaltend, konjugiert mit einem Reporter bzw. einem Quencher und RNase; und
(3) Nachweisen der aus dem Reporter erzeugten Fluoreszenz,
wobei die Nukleinsäure von Schritt (1) mit einem kationischen Phasentransferagens konjugiert wird, und
wobei die Nukleinsäure die Addition von Lipid am 3'-Ende und/oder am 5'-Ende aufweist.

2. Verfahren gemäß Anspruch 1, wobei die Nukleinsäure aus der Gruppe ausgewählt ist, die aus DNA, RNA und DNA-RNA-Hybrid besteht.

3. Verfahren gemäß Anspruch 1 oder Anspruch 2, wobei die Nukleinsäure Einzelstrang-DNA ist.

4. Verfahren gemäß Anspruch 1, wobei das Lipid C₇- -C₂₄-Lipid ist.

5. Verfahren gemäß Anspruch 1, wobei das Nukleinsäure enthaltende Objekt aus der Gruppe ausgewählt ist, die aus Benzin, Kerosin, Diesel, Farbe für Automobilbeschichtung, Lack, Farbe für Fahrspurmarkierung, Farbverdünner, Verdünnungsmittel, Schießpulver, Naturöl, Baufarbe, organischem Lösungsmittel, Klebstoff, Farbstoff, Fleisch und Meeresfrüchten besteht.

6. Verfahren gemäß Anspruch 1, wobei die Nukleinsäure die Nukleotidsequenz aufweist, welche 5 - 1000 Nukleotide beinhaltet.

7. Verfahren gemäß Anspruch 1, wobei die RNase RNase H ist.

8. Verfahren gemäß Anspruch 1, wobei der Reporter und der Quencher entweder mit dem 5'-Ende oder dem 3'-Ende der RNA-Dual-Sonde konjugiert sind.

9. Verfahren gemäß Anspruch 8, wobei der Reporter aus der Gruppe ausgewählt ist, die aus TAMRA (Carboxy-tetramethyl-rhod-amin), FAM (6-Carboxyfluorescein), Cy3, Cy5 und Cy5.5 besteht.

10. Verfahren gemäß Anspruch 8, wobei der Quencher aus der Gruppe ausgewählt ist, die aus BHQ1 (2,5-Di-tert-butylhydrochinon-1), BHQ2, TAMRA und DABCYL besteht.

11. Verfahren zum Identifizieren eines Nukleinsäure enthaltenden Objekts, das die folgenden Schritte beinhaltet:
(1) Vorbereiten des Nukleinsäure enthaltenden Objekts, mit der Nukleotidsequenz komplementär zur Nukleotidsequenz für RNA-Dual-Sonde;
(2) Rückgewinnen der Nukleinsäure aus dem Objekt;
(3) Reagieren der in Schritt (2) rückgewonnenen Nukleinsäure mit dem Puffer, die RNA-Dual-Sonde enthaltend, konjugiert mit einem Reporter bzw. einem Quencher und RNase; und
(4) Nachweisen der aus dem Reporter erzeugten Fluoreszenz,
wobei die Nukleinsäure von Schritt (1) mit einem kationischen Phasentransferagens konjugiert wird, und
wobei die Nukleinsäure die Addition von Lipid am 3'-Ende und/oder am 5'-Ende aufweist.

12. Verfahren gemäß Anspruch 1, wobei der Puffer zusätzlich RNase-Inhibitor enthält.

13. Verfahren zum Bestätigen, ob ein Nukleinsäure enthaltendes Objekt echt ist, das die folgenden Schritte beinhaltet:
(1) Vorbereiten des Nukleinsäure enthaltenden Objekts, mit der Nukleotidsequenz komplementär zur Nukleotidsequenz für RNA-Dual-Sonde;
(2) Reagieren der im Objekt eingeschlossenen Nukleinsäure mit dem Puffer, die RNA-Dual-Sonde enthaltend, konjugiert mit einem Reporter bzw. einem Quencher und RNase; und
(3) Nachweisen der aus dem Reporter erzeugten Fluoreszenz,
wobei die Nukleinsäure von Schritt (1) mit einem kationischen Phasentransferagens konjugiert wird, und
wobei die Nukleinsäure die Addition von Lipid am 3'-Ende und/oder am 5'-Ende aufweist.

14. Verfahren gemäß Anspruch 13, wobei das Nukleinsäure enthaltende Objekt aus der Gruppe ausgewählt ist, die aus Benzin, Kerosin, Diesel, Farbe für Automobilbeschichtung, Lack, Farbe für Fahrspurmarkierung, Farbverdünner, Verdünnungsmittel, Schießpulver, Naturöl, Baufarbe, organischem Lösungsmittel, Klebstoff, Farbstoff, Fleisch und Meeresfrüchten besteht.

## Revendications

1. Procédé d'identification d'un objet contenant un acide nucléique,
comprenant les étapes suivantes de :
(1) préparation d'un objet contenant un acide nucléique possédant la séquence nucléotidique complémentaire à la séquence nucléotidique d'une sonde ARN double ;
(2) mise en réaction de l'acide nucléique compris dans l'objet avec le tampon contenant la sonde ARN double conjuguée à un rapporteur et à un extincteur, respectivement, et une RNase ; et
(3) détection de la fluorescence générée à partir du rapporteur,
ledit acide nucléique de l'étape (1) étant conjugué à un agent de transfert de phase cationique, et
ledit acide nucléique présentant l'addition d'un lipide à l'extrémité 3' et/ou à l'extrémité 5'.

2. Procédé selon la revendication 1, ledit acide nucléique étant choisi dans le groupe constitué par un ADN, un ARN, et un hybride ADN-ARN.

3. Procédé selon la revendication 1 ou 2, ledit acide nucléique étant un ADN simple brin.

4. Procédé selon la revendication 1, ledit lipide étant un lipide en C₇ à C₂₄.

5. Procédé selon la revendication 1, ledit objet contenant un acide nucléique étant choisi dans le groupe constitué par l'essence, le kérosène, le diesel, une peinture pour revêtement automobile, un vernis, une peinture pour indication de voies de circulation, un diluant de peinture, un épaississant, une poudre à canon, une huile naturelle, une peinture pour construction, un solvant organique, une colle, un colorant, une viande et des fruits de mer.

6. Procédé selon la revendication 1, ledit acide nucléique ayant une séquence nucléotidique comprenant 5 à 1000 nucléotides.

7. Procédé selon la revendication 1, ladite RNase étant la RNase H.

8. Procédé selon la revendication 1, le rapporteur et l'extincteur étant conjugués soit à l'extrémité 5', soit à l'extrémité 3' de la sonde ARN double.

9. Procédé selon la revendication 8, ledit rapporteur étant choisi dans le groupe constitué par la TAMRA (carboxy-tétraméthyl-rhodamine), la FAM (6-carboxyfluorescéine), la Cy3, la Cy5 et la Cy5.5.

10. Procédé selon la revendication 8, ledit extincteur étant choisi dans le groupe constitué par la BHQ1 (2,5-di-tert-butylhydroquinone-1), la BHQ2, la TAMRA et le DABCYL.

11. Procédé d'identification d'un objet contenant un acide nucléique,
comprenant les étapes suivantes de :
(1) préparation d'un objet contenant un acide nucléique possédant la séquence nucléotidique complémentaire à la séquence nucléotidique d'une sonde ARN double ;
(2) récupération de l'acide nucléique à partir de l'objet ;
(3) mise en réaction de l'acide nucléique récupéré dans l'étape (2) avec le tampon contenant la sonde ARN double conjuguée à un rapporteur et à un extincteur, respectivement, et une RNase ; et
(4) détection de la fluorescence générée à partir du rapporteur,
ledit acide nucléique de l'étape (1) étant conjugué à un agent de transfert de phase cationique, et
ledit acide nucléique présentant l'addition d'un lipide à l'extrémité 3' et/ou à l'extrémité 5'.

12. Procédé selon la revendication 1, ledit tampon contenant en outre un inhibiteur de RNase.

13. Procédé de confirmation pour savoir si un objet contenant un acide
nucléique est authentique, comprenant les étapes suivantes de :
(1) préparation d'un objet contenant un acide nucléique possédant la séquence nucléotidique complémentaire à la séquence nucléotidique d'une sonde ARN double ;
(2) mise en réaction de l'acide nucléique compris dans l'objet avec le tampon contenant la sonde ARN double conjuguée à un rapporteur et à un extincteur, respectivement, et une RNase ; et
(3) détection de la fluorescence générée à partir du rapporteur,
ledit acide nucléique de l'étape (1) étant conjugué à un agent de transfert de phase cationique, et
ledit acide nucléique présentant l'addition d'un lipide à l'extrémité 3' et/ou à l'extrémité 5'.

14. Procédé selon la revendication 13, ledit objet contenant un acide nucléique étant choisi dans le groupe constitué par l'essence, le kérosène, le diesel, une peinture pour revêtement automobile, un vernis, une peinture pour indication de voies de circulation, un diluant de peinture, un épaississant, une poudre à canon, une huile naturelle, une peinture pour construction, un solvant organique, une colle, un colorant, une viande et des fruits de mer.
